# EUROPEAN PATENT APPLICATION

(11) **EP 3 257 502 A1**
(43) Date of publication of application: **20.12.2017**
(21) Application number: 16175032.8
(22) Date of filing: 17.06.2016
(51) Int. Cl.: A61K 9/48, A61K 9/52, A61K 31/7076, A61K 45/06, A61P 25/24, A61P 1/16, A61P 9/00, A61P 25/02, A61P 27/02

(54) **FORMULATIONS IN THE FORM OF CAPSULES FOR ORAL SYSTEMIC ADMINISTRATION COMPRISING S-ADENOSYLMETHIONINE AND POLYUNSATURATED FATTY ACIDS, PREPARATION AND USE THEREOF**

(71) Applicant: Stramentinoli, Giorgio, 20141 Milano (IT); Busetti, Cesare, 20149 Milano (IT)
(72) Inventor: Stramentinoli, Giorgio, 20141 Milano (IT); Busetti, Cesare, 20149 Milano (IT)
(74) Representative: Brighenti, Livio

(57) **Abstract**

There are described formulations in the form of capsules for oral systemic administration comprising S-adenosylmethionine or salts thereof as an active ingredient, wherein said active ingredient is dispersed in a lipid carrier selected from: omega-3 or omega-6 fatty acids.

## Description

### Field of the invention.

The present invention relates to the field of pharmaceutical and/or nutritional formulations for oral use, in particular formulations in the form of capsules containing S-adenosylmethionine and polyunsaturated fatty acids.

### Prior art

One of the problems of formulations containing S-adenosylmethionine (hereinafter, SAMe) is the preservation of the active ingredient from the destabilizing action of humidity, without affecting the effectiveness of its action.

In order achieve the above objectives, the pharmaceutical form currently most used both in Europe and in the USA is the gastro-resistant tablet.

However, the processing method underlying the production of gastro-resistant tablets is complex, both due to the hygroscopicity of the active ingredient and its concentration within the tablet, and this of course results in a high production cost that impacts heavily on the cost of the finished product, already burdened by that not negligible of the active ingredient.

The concentration of the active ingredient and the gastro-resistance also affect the tablet dissolution time and the absorption of the active ingredient.

Therefore, the importance of a formulation that allows a prolonged storage of the active ingredient while not interfering or if possible increasing its efficacy when administered to the patient is clear.

### Summary of the invention

There are described formulations in the form of capsules for oral systemic administration comprising S-adenosylmethionine or salts thereof as an active ingredient, wherein said active ingredient is dispersed in a lipid carrier.

### Detailed description of the invention

The present invention allows to overcome the aforementioned problems with formulations in the form of capsules, either soft or hard, in which in order to isolate the active ingredient (SAMe or salts thereof) from the environment and capsule humidity, this is first dispersed in a lipid carrier.

The active ingredient, SAMe, may be present as such or in the form of salt, such as SAMe tosylate, SAMe sulfate, SAMe 1,4 butanedisulfonate.

By lipid carrier it is meant any lipid substance of natural origin.

The lipid carrier may consist of medium and long chain fatty acid triglycerides, and/or vegetable and animal waxes, such as beeswax, and/or terpene hydrocarbons, of vegetable origin, such as squalene or squalane.

The medium and long chain fatty acid triglycerides are of vegetable origin, such as sesame oil, sweet almond oil, coconut oil, corn oil, olive oil, castor oil, jojoba oil, and can be used in their natural state or partially or fully hydrogenated, such as Cutin HR^{®} Powder by Basf.

More in particular, an active lipid carrier optionally admixed with other excipients is preferred among the lipid substances.

By active lipid carrier it is meant a lipid component having its own defined, clinically proven pharmacological activity, such as polyunsaturated fatty acids; particularly preferred are omega-3 fatty acids, also known as PUFAS n-3, such as for example α-linolenic acid (ALA), eicosapentaenoic acid (EPA) or docosahexaenoic acid (DHA) and omega-6 fatty acids, such as γ-linolenic acid (GLA). The active lipid components can be of vegetable origin, such as flaxseed oil, borage oil, soybean oil, or be of animal origin, such as fish or krill oil.

The above excipients normally used can be divalent metal salts, such as magnesium and calcium, or monovalent, such as sodium or mixtures thereof. Magnesium sulfate, known as English salt, is particularly preferred.

Another excipient of potential interest is Polyglyceryl-3 dioleate (known as Plurol Oleique CC497^{®}, by Gattefossé), whose fluidizer action favors dosage at higher concentrations.

Normally, in the capsules according to the invention the active ingredient is 25-65% by weight of the total content of the capsule, the active lipid carrier is present up to 70% by weight of the total content of the capsule and the excipients are present up to 15% by weight on the content of the capsule.

Moreover, if preferred, the capsules may contain other active ingredients, such as for example antioxidants, vitamins, inositol, 5-HTP, amino acids, folic acid and folate, minerals, such as selenium, zinc, magnesium, copper, iodine, either in the form of salt or in the form of liposomes or complexes.

The capsules according to the invention may be prepared with the standard methodology known as *"Liquid Fill Hard Capsule Manufacturing*".

For example, the SAMe is added to the lipid carrier, if needed previously heated to about 50 °C and it is kept under stirring until complete mixing.

The mixture is dosed into capsules, such as Licaps® capsules by Capsugel in various formats and weight, such as capsules in formats of between 1 and 000 and weight of between 450 and 900 mg.

After dosing, the capsules are sealed by means of a banding machine and filmed in a coating pan using a gastro-resistant film.

Filming is carried out with the use of conventional methodologies and standard gastro-resistant polymers, such as hydroxypropyl methyl ethyl cellulose phthalate (HPMCP), hydroxypropyl methyl ethyl cellulose acetate succinate (HPMCAS), cellulose acetate phthalate (CAP), such as Aquacoat^{®} CPD by FMC, shellac, mixture of ethyl cellulose (EC) and NS Enteric^{®}, such as Nutrateric^{®} by Colorcon, polyacrylates/acrylic resins, such as Eudragit^{®} L30 D-55 by Evonik or Kollicoat^{®} MAE 30 D by Basf, or modified corn starch, such as Amprac 01 by Rofarma Italy. The capsules according to the invention may be used for treating depression, liver diseases, inflammatory diseases and peripheral neuropathy (diabetic and non-diabetic), and microvascular disorders of the retina.

### Example 1

Components:

| | |
|---|---|
| Lipid carrier | 65% |
| SAMe | 35% |

The lipid carrier may consist of vegetable oils, animal oils, medium and long chain triglycerides, etc.

The preparation is done by mixing the SAMe in lipid carrier and dosing the resulting mixture in the capsules that are made gastro-resistant by filming.

### Example 2

Components:

| | |
|---|---|
| Vegetable DHA | 350 mg |

| | |
|---|---|
| SAMe | 300 mg |
| Borage oil | 100 mg |

The mixture was processed as described in example 1 and was dosed into hard gelatin capsules, format 0el, to a weight of 750 mg and then sealed and filmed with a gastro-resistant layer.

### Example 3

### Industrial test

| | | |
|---|---|---|
| Components: | | |
| SAMe | 400 mg | |
| Omevital 1812 TG Gold | 482 mg | (Basf) |
| Aerosil 200 | 18 mg | (Evonik) |

Operating parameters:

| | |
|---|---|
| process type: | Capsule filling with liquid |
| equipment used: | Zanasi Plus 40 E |
| capsule format: | 00 LT-Capsugel |
| empty capsule weight: | 120 mg ± 3% |
| production speed: | 13800 cps/hour |
| tank temperature: | 50 ± 2 °C |

The capsules were sealed with a "Capsule Banding Machine" Hermetica by IMA and filmed in a perforated basket coating pan, applying 45 mg/cps of Nutrateric^{®} (Colorcon).

The capsules were subjected to preliminary stability study in different packaging materials.

### Example 4

| | | |
|---|---|---|
| SAMe | 200 mg | |
| Omevital 1812 TG Gold | 300 mg | (Basf) |
| Magnesium sulfate anhydrous | 30 mg | |
| Silicon dioxide | 20 mg | (Sipernat 50S - Evonik) |

The mixture was dosed into HPMC capsules, format 0, to a weight of 550 mg and then sealed and filmed with a gastro-resistant layer.

### Example 5

Components:

| | |
|---|---|
| SAMe | 400 mg |
| DHA | 400 mg |
| Magnesium sulfate anhydrous | 30 mg |
| Polyglyceryl-3 dioleate | 40 mg |

The mixture was dosed into hard gelatin capsules, format 00, to a weight of 870 mg and then sealed with a gelatin solution and filmed with a gastro-resistant layer.

### Example 6

Components:

| | |
|---|---|
| Medium chain triglycerides | 450 mg |
| Glyceryl dibehenate | 50 mg |
| SAMe | 300 mg |

Medium chain triglycerides (C12-C18, commercially known, for example, by the name of Labrafac Lipophile WL 1349 by Gattefossé) and glyceryl dibehenate (commercially known as Compritol E ATO by Gattefossé) are melted in a thermostat-controlled container at about 80 °C. After melting, the mixture is kept under mild stirring and cooled to about 50 °C.

Upon reaching this temperature, the SAMe is added, then the product is regulated, under stirring, to about 45-50 °C for the entire processing time.

The mixture is dosed into vegetable capsules DRcaps™ by Capsugel, format 00 to the weight of 800 mg/capsule.

After the dosage in capsules, as a result of the reduction in temperature and in the absence of stirring, the suspension hardens, thereby blocking the SAMe in a lipid matrix that does not require subsequent sealing or gastro-resistant filming.

### Example 7

Soft gelatin capsules - Industrial test of 30,000 capsules

Components for one capsule:

| | | |
|---|---|---|
| SAMe | 400 | mg |
| Fish oil EE 10/50 | 532 | mg, of which: |
| • | EPA 53.2 | mg |
| • | DHA 250 | mg |
| Magnesium sulfate anhydrous | 20 | mg |

Mono- and diglycerides of fatty acids as needed
Sunflower lecithin as needed

Heat a part of the oil (about one quarter of the total) to 65 °C, then add the mono and diglycerides of fatty acids and the sunflower lecithin. Mix until complete melting of the components. Add another part of oil and cool to 45 °C. Upon reaching this temperature, add the SAMe and magnesium sulfate anhydrous and mix until completely homogeneous. Add the remaining oil, mix and bleed.

The mixture is dosed into soft gelatin capsules, format 16 oblong, brick colored and subjected to humidity-controlled chamber drying.

The dried soft capsules are placed in a coating pan and made gastro-resistant with a suspension of Nutrateric® (Colorcon Limited).

### Example 8

### STABILITY DATA OF SAME/OMEGA-3 ENTERIC COATED SOFTGEL CAPSULES

### BATCH Nr. D093D

### CONDITIONS: 5°C±3°C

| | **Initial** | **T15 days** | **T30 days** | **T90 days** | **T180 days** |
|---|---|---|---|---|---|
| Appearance | Softgel | C | C | C | C |
| Color | Brick red | C | C | C | C |
| Odor | Characteristic | C | C | C | C |
| Flavor | Characteristic | C | C | C | C |
| SAMe ion HPLC% | 100,00 | 100,23 | 100,03 | 99,98 | 99,86 |
| Adenosine | 0,00 | 0,01 | 0,01 | 0,01 | 0,01 |
| Adenine | 0,07 | 0,07 | 0,08 | 0,09 | 0,10 |
| S.Ad.Homocyst. | 0,07 | 0,06 | 0,06 | 0,08 | 0,08 |
| MTA | 0,04 | 0,04 | 0,05 | 0,06 | 0,06 |
| dcSAM | 0,24 | 0,24 | 0,23 | 0,21 | 0,18 |
| Unknown | <0,1 | <0,1 | <0,1 | <0,1 | <0,1 |
| Total Impurities | 0,51 | 0,28 | 0,51 | 0,56 | 0,59 |
| | | | | | |
| C=Conform | | | | | |

### STABILITY DATA OF SAME/OMEGA-3 ENTERIC COATED SOFTGEL CAPSULES

### BATCH Nr. D093D

### CONDITIONS: 25°C/60% RH

| | **Initial** | **T15 days** | **T30 days** | **T90 days** | **T180 days** |
|---|---|---|---|---|---|
| Appearance | Softgel | C | C | C | C |
| Color | Brick red | C | C | C | C |
| Odor | Characteristic | C | C | C | C |
| Flavor | Characteristic | C | C | C | C |
| SAMe ion HPLC% | 100,00 | 100,10 | 99,10 | 97,07 | 92,44 |
| Adenosine | 0,00 | 0,01 | 0,01 | <LOQ | <LOQ |
| Adenine | 0,07 | 0,12 | 0,24 | 0,60 | 1,30 |
| S.Ad.Homocyst. | 0,07 | 0,05 | 0,05 | 0,08 | 0,11 |
| MTA | 0,04 | <LOQ | 0,03 | 0,05 | 0,03 |
| dcSAM | 0,24 | 0,24 | 0,23 | 0,21 | 0,18 |
| Unknown | <0,1 | <0,1 | <0,1 | <0,1 | <0,1 |
| Total Impurities | 0,51 | 0,24 | 0,65 | 1,13 | 2,02 |
| | | | | | |
| C=Conform | | | | | |

## Claims

1. Formulations in the form of capsules comprising SAMe or salts thereof as active ingredient, and bivalent or monovalent metal salts or mixtures thereof as excipients, wherein said active ingredient is dispersed in a lipid carrier selected from: omega-3 or omega-6 fatty acids.

2. Formulations according to claim 1, wherein the excipient is magnesium sulfate.

3. Formulations according to claims 1 and 2, wherein the active ingredient is 25-65% by weight, the active lipid carrier is present up to 70% by weight, and the excipients are present up to 15% by weight, wherein said percentages are calculated on the total content of the capsule.

4. Formulations according to claims 1-3, further containing: antioxidants, vitamins, inositol, 5-HTP, amino acids, folic acid and folate, minerals, such as selenium, zinc, magnesium, copper, iodine, either in the form of salt or in the form of liposomes or complexes.

5. Formulations according to claims 1-4, in the form of hard or soft capsules.

6. A process for preparing capsules according to claims 1-5, wherein:
- SAMe is mixed with the lipid carrier and with any excipients,
- the mixture is kept under stirring, for all the processing time,
- the mixture is dosed in capsules of various format and weight,
- the capsules are sealed,
- the capsules are filmed with a gastro-resistant film.

7. Capsules according to claims 1-5 for use in the treatment of depression, liver diseases, inflammatory diseases and peripheral (diabetic and non-diabetic) neuropathies, and microvascular retinal disorders.
